# FASCICULE DE BREVET EUROPEEN

(11) **EP 0 958 856 B1**
(45) Date de publication et mention de la délivrance du brevet: **02.07.2003**
(21) Numéro de dépôt: 99400923.1
(22) Date de dépôt: 15.04.1999
(51) Int. Cl.: B01F 17/00, A61K 7/00

(54) **Emulsion E/H/E stable et son utilisation comme composition cosmétique et/ou dermatologique**
Dreifachemulsion W/O/W und ihre Verwendung für kosmetische und/oder dermatologische Zusammensetzung
W/O/W triple emulsion and its use for cosmetic and/or dermatologic composition

(30) Priorité: 20.05.1998 FR 9806412
(43) Date de publication de la demande: 24.11.1999
(73) Titulaire: L'OREAL, 75008 Paris (FR)
(72) Inventeur: Simon, Pascal, 94400 Viytry/S/Seine (FR)
(74) Mandataire: Rasson, Catherine

(56) Documents cités:
- EP-A- 0 545 002
- EP-A- 0 631 774
- EP-A- 0 780 114
- FR-A- 2 693 466

## Description

La présente invention se rapporte à une émulsion triple eau/huile/eau stable et à son utilisation notamment dans les domaines cosmétique et/ou dermatologique, en particulier pour la libération contrôlée d'actifs, en vue notamment de nettoyer et/ou traiter et/ou protéger la peau et/ou les muqueuses et/ou les fibres kératiniques.

Il est connu d'utiliser, notamment dans les domaines cosmétique et dermatologique, des compositions topiques sous forme d'émulsions. Ces émulsions sont généralement des émulsions huile-dans-eau (H/E) ou eau-dans-huile (E/H). Il peut s'agir aussi d'émulsions multiples du type eau/huile/eau (E/H/E) ou huile/eau/huile (H/E/H). Parmi les émulsions multiples, on utilise de préférence les émulsions à phase aqueuse externe, à savoir les émulsions E/H/E, qui allient les avantages de la fraîcheur à l'application, apportée par l'eau présente dans la phase externe aqueuse, et du confort apporté par une quantité relativement importante d'huile.

Toutefois, les émulsions multiples sont peu exploitées car elles présentent fréquemment des problèmes de stabilité dans le temps. Le mécanisme de déstabilisation le plus fréquemment rencontré est la migration d'eau des gouttelettes internes vers le milieu aqueux externe à travers la couche huileuse intermédiaire, soit par simple diffusion d'eau à travers la membrane huileuse, soit par rupture préalable du film huileux provoquant la coalescence des gouttelettes d'eau internes et conduisant à un relargage d'eau interne dans le milieu aqueux externe. Généralement ce phénomène dit de perte du caractère multiple finit par aboutir à un déphasage visible macroscopiquement et à l'obtention d'une émulsion H/E simple au lieu d'une émulsion triple.

Aussi, différents moyens ont été envisagés pour pallier à cet inconvénient. En particulier, une des solutions consiste à introduire dans la phase aqueuse interne ou dans la phase aqueuse externe un ou plusieurs polymère(s) gélifiant(s) dont le rôle est de limiter durablement les mouvements d'eau de la phase interne vers la phase externe. Toutefois, les émulsions multiples obtenues présentent le défaut d'être collantes et longues à pénétrer dans la peau à cause de la présence de fortes quantités de polymères qui restent à la surface de la peau du fait de leur structure polymérique.

Une autre solution consiste à introduire des agents structurants lipophiles dans la phase huileuse. Ainsi, le document FR-A-2,679,788 préconise l'utilisation d'alcools gras linéaires insaturés en C₈ à C₁₄ donnant lieu à des savons et conférant à l'émulsion une structure gélifiée. Malheureusement, les qualités sensorielles des émulsions obtenues sont peu satisfaisantes car elles ont tendance à devenir cireuses à l'application sur la peau. En outre, la présence de savons dans l'émulsion a pour conséquence un phénomène de savonnage, c'est-à-dire de blanchiment lors de l'application sur la peau.

En outre, il est connu par exemple par le document WO-A-94/1073 d'utiliser dans les émulsions triples, des polymères de silicone comportant une chaîne polyoxyéthylénée et/ou polyoxypropylénée, qui, bien que polymères, n'ont pas les inconvénients cités ci-dessus. Toutefois, l'emploi de ce type d'émulsifiant nécessite la présence d'une certaine quantité d'huile de silicone dans la phase huileuse et limite donc la composition galénique de l'émulsion triple. En outre, pour obtenir une stabilité satisfaisante de l'émulsion triple, il est souvent nécessaire d'ajouter un agent gélifiant dans une des phases aqueuses.

Il subsiste donc le besoin d'une émulsion multiple E/H/E stable n'ayant pas les inconvénients de celles de l'art antérieur et étant notamment agréable à utiliser sur la peau en apportant, par exemple, les avantages d'une émulsion à phase aqueuse externe.

La demanderesse a maintenant trouvé de manière inattendue que l'introduction d'un organopolysiloxane élastomère partiellement ou totalement réticulé comportant une chaîne polyoxyéthyléné et/ou polyoxypropyléné dans une émulsion triple eau/huile/eau (E/H/E) permettait de stabiliser la dite émulsion sans nécessiter l'ajout d'autres agents stabilisants.

Aussi, la présente invention a pour objet une émulsion triple eau/huile/eau comportant une phase aqueuse externe et une émulsion primaire E/H comprenant une phase huileuse et une phase aqueuse interne, caractérisée en ce qu'elle contient au moins un organopolysiloxane élastomère partiellement ou totalement réticulé comportant une chaîne polyoxyéthylénée et/ou polyoxypropylénée, l'organopolysiloxane élastomère étant défini ci-dessous.

La présente invention a aussi pour objet l'utilisation d'au moins un organopolysiloxane élastomère partiellement ou totalement réticulé comportant une chaîne polyoxyéthylénée et/ou polyoxypropylénée pour la stabilisation d'une émulsion triple eau/huile/eau, l'orgamopolysiloxane élastomère étant défini ci-dessous.

Pour une application topique, l'émulsion selon l'invention doit contenir un milieu topiquement acceptable, c'est-à-dire compatible avec la peau, les muqueuses et/ou les fibres kératiniques telles que les cheveux.

L'émulsion triple selon l'invention a l'avantage d'être stable et de pouvoir notamment préserver l'activité d'actifs présents dans la phase aqueuse interne, d'où ils sont libérés lors de l'application de la composition sur la peau, les muqueuses et/ou les cheveux.

Les organopolysiloxanes élastomères partiellement ou totalement réticulés utilisables dans l'émulsion selon la présente invention sont introduits dans la phase huileuse de l'émulsion. Ce sont généralement des émulsifiants. Ils sont choisis notamment parmi les polymères réticulés décrits dans la demande EP-A-0545002 indiquée ici par référence. Ces organopolysiloxanes sont obtenus par polymérisation d'addition des composés (I) et (II) suivants :
(I) un organohydrogènepolysiloxane de formule (1) :

   R¹ ₐR² _{b}H_{c}SiO_{(4-a-b-c)/2} (1)

   dans laquelle R¹ représente un groupe alkyle, aryle ou aralkyle substitué ou non substitué, comportant de 1 à 18 atomes de carbone, ou un groupe hydrocarboné halogéné ; R² représente un groupe :

   -CₙH₂ₙO(C₂H₄O)_{d}(C₃H₆O)ₑR³ (3)

   dans lequel R³ est un hydrogène, un groupe hydrocarboné aliphatique saturé ayant de 1 à 10 atomes de carbone ou un groupe -(CO)-R⁵ où R⁵ est un groupe hydrocarboné aliphatique saturé ayant de 1 à 5 atomes de carbone; d est un nombre entier de 2 à 200 et e est un nombre entier de 0 à 200, pourvu que d + e soit un nombre allant de 3 à 200, et n est un nombre de 2 à 6, a est une valeur satisfaisant à l'inégalité : 1.0 ≤ a ≤ 2.5, b est une valeur satisfaisant à l'inégalité : 0.001 ≤ b ≤ 1.0 et c est une valeur satisfaisant à l'inégalité : 0.001 ≤ c ≤ 1.0 ;
   ou un organohydrogènepolysiloxane représenté par la formule (2) suivante :

   R¹ _{f}H_{g}SiO_{(4-f-g)/2} (2)

   dans laquelle R¹ a la même signification que dans la formule (1), f est une valeur satisfaisant à l'inégalité : 1.0 ≤ f ≤ 3.0, g est une valeur satisfaisant à l'inégalité : 0.001 ≤ g ≤ 1.5 ;
   ou un mélange des organohydrogènepolysiloxanes de formules (1) et (2), et
(II) un polyoxyalkylène représenté par la formule (A) suivante :

   CₘH₂ₘO(C₂H₄O)ₕ(C₃H₆O)ᵢCₘH₂ₘ₋₁ (A)

   dans laquelle h est un nombre entier allant de 2 à 200, i est un nombre entier allant de 0 à 200, pourvu que h + i soit un nombre allant de 3 à 200, et m est un nombre allant de 2 à 6,
ou un organopolysiloxane représenté par la formule (B) suivante :

R¹ ⱼR⁴ ₖSiO_{(4-j-k)/2} (B)

dans laquelle R¹ a la même signification que dans la formule (1), R⁴ est un groupe hydrocarboné monovalent ayant une liaison aliphatique insaturée en extrémité et contenant 2 à 10 atomes de carbone, j est une valeur satisfaisant à l'inégalité : 1.0 ≤ j ≤ 3.0 et k est une valeur satisfaisant à l'inégalité : 0.001 ≤ k ≤ 1.5,
ou un mélange du polyoxyalkylène de formule (A) et de l'organopolysiloxane de formule (B), où au moins un organohydrogènepolysiloxane de formule (1) ou au moins un polyoxyalkylène de formule (A) est contenu comme élément essentiel de la polymérisation d'addition.

De manière préférée, l'organopolysiloxane est en mélange avec une huile de silicone et/ou un polyol et est préparé directement en un tel mélange. L'huile de silicone présente de préférence une viscosité égale ou inférieure à 100 cSt à 25°C. Selon un mode de réalisation de l'invention, l'organopolysiloxane élastomère est préparé à partir de 100 parties en poids des constituants définis ci-dessus et 3 à 200 parties en poids d'une huile de silicone ayant une viscosité égale ou inférieure à 100 mm²/s à 25°C, et/ou d'un polyol. L'huile de silicone peut être une huile de silicone volatile ou non volatile ou un mélange d'huile de silicone volatile et d'huile de silicone non volatile.

Les organopolysiloxanes de l'invention sont en particulier obtenus selon le mode opératoire des exemples 3, 4 et 8 du document EP-A-545002 (ou US-5,412,004) et des exemples du document US-A-5,811,487.

Les organopolysiloxanes de la composition de l'invention contiennent un ou plusieurs groupements oxyalkylénés et en particulier oxyéthylénés (OE), par exemple de 1 à 40 motifs oxyalkylénés et mieux 1 à 20 motifs oxyalkylénés, pouvant former des chaînes polyoxyalkylènes et notamment polyoxyéthylènes. Ces groupements peuvent être pendants, en bout de chaîne ou destinés à lier deux parties de la structure siliconée. Les atomes de silicium portant ces groupements sont au nombre d'environ 1 à 10.

Bien que l'invention concerne plus spécialement les organopolysiloxanes à groupement(s) oxyéthyléné(s), elle peut aussi concerner les organopolysiloxanes à groupement(s) oxypropyléné(s). Les organopolysiloxanes peuvent aussi comporter à la fois un ou plusieurs groupement(s) oxyéthyléné(s), 1 à 20 (OE) par exemple, et un ou plusieurs groupement(s) oxypropyléné(s) (OP), 0 à 20 par exemple ; ces organopolysiloxanes sont appelés aussi des organopolysiloxanes à groupement(s) alkyléthoxy-propyléné(s). De préférence, le nombre de groupements oxyéthylénés est supérieur au nombre de groupements oxypropylénés.

Comme organopolysiloxane partiellement ou totalement réticulé comportant une chaîne polyoxyéthylénée et/ou polyoxypropylénée, on peut citer par exemple le produit commercialisé par Shin-Etsu sous la dénomination de KSG21. Ce produit comprend 38 % d'organopolysiloxane et 62 % d'huile de silicone ayant une viscosité de 6 cSt. On peut citer aussi le produit de l'exemple 3 du brevet US-5,412,004, contenant environ 33 % en poids d'organopolysiloxane et environ 67 % d'huile de silicone ayant une viscosité de 6 cSt.

Dans l'émulsion triple selon l'invention, l'organopolysiloxane partiellement ou totalement réticulé est utilisé de préférence en une quantité en matière active allant de 0,1 à 10 % et de préférence de 1 à 5 % du poids total de l'émulsion triple.

De préférence, la phase aqueuse externe de l'émulsion triple contient au moins un tensioactif non ionique ayant un HLB (Balance Hydrophile Lipophile) supérieur à 12 et l'émulsion primaire contient au moins un tensioactif non ionique ayant un HLB inférieur à 8.

Le tensioactif non ionique de HLB > 12 éventuellement présent dans l'émulsion triple peut être notamment choisi parmi les alcools gras éthoxylés ou éthoxylés/propoxylés à chaine grasse comportant de 12 à 22 atomes de carbone, les stérols éthoxylés tels que le PEG-16 soya stérol ou le PEG-10 soya stérol, les copolymères blocs d'oxyde d'éthylène et d'oxyde de propylène (Poloxamer), et leurs mélanges. On utilise de manière préférée les stérols éthoxylés et les poloxamers.

La quantité de tensioactif dans la phase aqueuse externe peut aller par exemple de 0,5 à 5 % et de préférence de 1 à 3 % du poids total de l'émulsion triple.

Le tensioactif non ionique de HLB < 8 peut être notamment choisi parmi les esters de glycéryle tels que les mono-, di- ou tri-isostéarate -ou -oléate de mono-, di- ou tri-glycéryle, les esters de sucre tels que le mono- ou di-isostéarate ou -oléate de sucrose ou de méthylglucose, les éthers d'alkyl-polyglucoside tels que l'oléyl- ou isostéaryl-polyglucoside, et leurs mélanges. On utilise de manière préférée les esters de sucre et les éthers d'alkyl-polyglucoside.

La quantité de tensioactif dans l'émulsion primaire selon l'invention peut aller, par exemple, de 0,01 à 5 % et de préférence de 0,5 à 3 % du poids total de l'émulsion triple.

La phase huileuse de l'émulsion primaire comprend un ou plusieurs corps gras choisis parmi les huiles d'origine animale, les huiles d'origine végétale (huile d'amande d'abricot), les huiles minérales (huile de vaseline), les huiles synthétiques (isohexadécane), les huiles fluorées, les cires et notamment les cires de silicone, les gommes de silicone, les résines de silicone.

La quantité de corps gras va de préférence de 2 à 40 % et mieux de 5 à 30 % du poids total de l'émulsion triple.

L'émulsion primaire peut représenter par exemple de 5 à 70 % et de préférence de 10 à 65 % du poids total de l'émulsion triple.

L'émulsion triple est préparée de manière classique par préparation de l'émulsion primaire et incorporation d'une quantité déterminée de l'émulsion primaire dans la phase aqueuse externe.

Comme indiqué en début de description, l'un des intérêts majeurs de l'émulsion conforme à l'invention est que cette dernière peut contenir, tout en présentant un caractère stable, des principes actifs, tant cosmétiques que thérapeutiques, ces actifs pouvant donc être notamment choisis parmi tous ceux habituellement utilisés à ce jour dans le domaine de la cosmétique, de la dermatologie ou du médicament.

L'invention a donc aussi pour objet une composition topique, caractérisée en ce qu'elle contient une émulsion telle que définie ci-dessus et au moins un actif.

Comme actifs, on peut citer notamment les polyols tels que la glycérine, les glycols et les dérivés de sucre, les bêta-hydroxyacides tels que l'acide salicylique et ses dérivés, les alpha-hydroxyacides tels que l'acide lactique et l'acide glycolique, les filtres, les hydratants tels que les hydrolysats de protéines, les vitamines telles que la vitamine E et leurs mélanges.

La composition de l'invention permet en outre de stabiliser tout actif instable en milieu oxydant, et on peut citer notamment comme actifs instables en milieu oxydant, certaines vitamines et notamment l'acide ascorbique (vitamine C) et ses dérivés, notamment ses dérivés glycosylés et phosphatés et ses esters comme l'acétate, le palmitate et le propionate d'ascorbyle, le rétinol (vitamine A) et ses dérivés, notamment ses esters comme l'acétate, le palmitate et le propionate de rétinol ; l'urée ; la rutine; les enzymes telles que la lipase, la protéase, la phospholipase et les cellulases ; les extraits naturels tels que le thé vert, l'extrait de mélisse, l'extrait de thym, les oligomères procyannidoliques (OPC) tels que l'OPC d'aubépine, l'OPC de pin et l'OPC de raisin ; certains acides tels que l'acide kojique, l'acide caféique, l'acide rétinoique et ses dérivés, l'acide benzène 1,4-di-(3-méthylidène 10-camphosulfonique) ; les caroténoïdes tels que les carotènes comme par exemple les α-, β- et γ-carotènes, le β,ϕ-carotène, le ξ-carotène, le β,λ-carotène, le lycopène (ψ,ψ-carotène) ; les acides gras polyinsaturés tels que l'acide gamma-linolénique, et leurs mélanges.

Il peut s'agir également de tous les composés naturels ou synthétiques pouvant contenir les actifs indiqués ci-dessus, en particulier les extraits végétaux, et plus spécialement les extraits de fruits.

La composition de l'invention est particulièrement intéressante pour stabiliser les vitamines, notamment la vitamine C et la vitamine A, et les caroténoïdes et notamment le lycopène.

La quantité d'actif dans la composition selon l'invention dépend du type d'actif utilisé et du but recherché. De manière générale, le ou les actifs peuvent être utilisés dans la composition selon l'invention en une quantité allant de 0,01 à 20 % en poids, de préférence de 0,04 à 15 %, et mieux de 0,1 à 10 % en poids par rapport au poids total de la composition.

Selon le caractère hydrophile ou lipophile des actifs utilisés, ceux-ci sont introduits dans la phase huileuse de la composition ou dans une des phases aqueuses, de préférence la phase aqueuse interne.

Les émulsions E/H/E selon l'invention peuvent être utilisées dans différentes applications topiques, notamment cosmétiques et/ou dermatologiques. La composition à base de cette émulsion peut constituer notamment des compositions de nettoyage, de protection, de traitement et/ou de soin de la peau, des muqueuses et/ou des cheveux, en particulier pour le visage, pour le cou, pour les mains, pour les cheveux, pour le cuir chevelu ou pour le corps, ainsi que pour les cils.

Aussi, l'invention a encore pour objet l'utilisation cosmétique de la composition selon l'invention pour nettoyer et/ou traiter et/ou protéger la peau et/ou les muqueuses et/ou les fibres kératiniques, c'est-à-dire les cheveux et/ou les cils.

L'invention a aussi pour objet l'utilisation de la composition selon l'invention pour la préparation d'une composition destinée à nettoyer et/ou traiter et/ou protéger la peau et/ou les muqueuses et/ou les fibres kératiniques, c'est-à-dire les cheveux et/ou les cils.

L'invention a aussi pour objet un procédé cosmétique pour nettoyer et/ou traiter et/ou protéger la peau, les muqueuses et/ou les fibres kératiniques, caractérisé en ce qu'il consiste à appliquer sur la peau, les muqueuses et/ou les fibres kératiniques, une composition telle que définie ci-dessus.

La composition selon l'invention peut constituer notamment des crèmes de protection, de traitement ou de soin pour le visage, pour les mains, pour les pieds, des laits corporels de protection ou de soin, des lotions, gels ou mousses pour le soin de la peau, des muqueuses, des cheveux et du cuir chevelu.

De façon connue, la composition de l'invention peut contenir également des adjuvants lipophiles ou hydrophiles habituels dans les domaines cosmétique et dermatologique, tels que les tensioactifs, notamment les tensioactifs moussants, les conservateurs, les antioxydants, les séquestrants, les solvants, les parfums, les charges, les filtres, les absorbeurs d'odeur, les matières colorantes et les vésicules lipidiques. Les quantités de ces différents adjuvants sont celles classiquement utilisées dans les domaines considérés, et par exemple de 0,01 à 15 % du poids total de la composition. Elle peut contenir également des vésicules lipidiques formées de lipides ioniques ou non ioniques.

Ces adjuvants, selon leur nature, peuvent être introduits dans la phase huileuse
ou dans une des phases aqueuses.

Les exemples ci-après de compositions selon l'invention, sont donnés à titre d'illustration et sans caractère limitatif. Les quantités y sont données en % en poids.

### Exemple 1 : Emulsion

### 1. Emulsion primaire :

### Phase A :

- Di-oléate de méthyl glucose 1,5 %
- Huile de vaseline 3,8 %
- Huile d'amande d'abricot 4,2 %
- Isohexadécane 3,6 %
- Cyclohexaméthylsiloxane 4,5 %
- KSG 21 (à 28% de M.A) 2,4 %

### Phase B :

- Sulfate de magnésium 0,4 %
- Glycérine 3 %
- Eau 39,2 %

### 2. Emulsion triple :

### Phase A :

- Emulsion primaire 62,6 %

### Phase B :

- Alcool cétylique 0,7 %
- PEG-10 soya stérol 2 %
- Conservateur 0,5 %
- Parfum 0,3 %
- Eau déminéralisée qsp 100 %

On a préparé une composition identique à celle de l'exemple 1 mais en remplaçant le KSG 21 par un dimethicone copolyol (à 10% dans du cyclomethicone).

L'émulsion obtenue est une émulsion simple et non une émulsion triple.

Ceci montre que seule l'association selon l'invention permet d'obtenir une émulsion triple stable.

### Exemple 2 : Emulsion

### 1. Emulsion primaire :

### Phase A :

- Di-oléate de méthyl glucose 1,5 %
- Huile de vaseline 3,8 %
- Huile d'amande d'abricot 4,2 %
- Isohexadécane 3,6 %
- Cyclohexaméthylsiloxane 4,5 %
- KSG 21 (à 28% de M.A) 2,4 %

### Phase B :

- Sulfate de magnésium 0,4 %
- Glycérine 10 %
- Vitamine C 3 %
- Eau 29,2 %

### 2. Emulsion triple :

### Phase A :

- Emulsion primaire 62,6 %

### Phase B :

- Alcool cétylique 0,7 %
- PEG-10 soya stérol 2 %
- Conservateur 0,5 %
- Parfum 0,3 %
- Eau déminéralisée qsp 100 %

### Exemple 3 : Emulsion

### 1. Emulsion primaire :

### Phase A :

- Huile de silicone 6 cSt 12 %
- Organosiloxane de l'exemple 3 du brevet US-5,412,004 18 % (soit environ 6 % de matière active)

### Phase B :

- Sulfate de magnésium 0,7 %
- Eau qsp 100 %

### 2. Emulsion triple :

### Phase A :

- Emulsion primaire 80 %

### Phase B :

- Poloxamer 407 (Synperonic PE/F 127 de la société ICI) 0,8 %
- Conservateur 0,5 %
- Eau déminéralisée qsp 100 %

On obtient une émulsion multiple stable.

### Exemple 4 : Emulsion

### 1. Emulsion primaire :

### Phase A :

- Huile de silicone 6 cSt 12 %
- Organosiloxane de l'exemple 3 du brevet US-5,412,004 18 % (soit environ 6 % de matière active)

### Phase B :

- Sulfate de magnésium 0,7 %
- Eau qsp 100 %

### 2. Emulsion triple :

### Phase A :

- Emulsion primaire 80 %

### Phase B :

- PEG-10 soya stérol 2 %
- Alcool cétylique 0,7 %
- Conservateur 0,5 %
- Eau déminéralisée qsp 100 %

On obtient une émulsion multiple stable.

## Revendications

1. Emulsion triple eau/huile/eau comportant une phase aqueuse externe et une émulsion primaire E/H comprenant une phase huileuse et une phase aqueuse interne, **caractérisée en ce que** la phase huileuse contient au moins un organopolysiloxane élastomère partiellement ou totalement réticulé comportant une chaîne polyoxyéthylénée et/ou polyoxypropylénée, obtenu par polymérisation d'addition des composés (I) et (II) suivants :
(I) un organohydrogènepolysiloxane de formule (1) :
R¹ ₐR² _{b}H_{c}SiO_{(4-a-b-c)/2} (1)
dans laquelle R¹ représente un groupe alkyle, aryle ou aralkyle substitué ou non substitué, comportant de 1 à 18 atomes de carbone, ou un groupe hydrocarboné halogène ; R² représente un groupe :
-CₙH₂ₙO(C₂H₄O)_{d}(C₃H₆O)ₑR³ (3)
dans lequel R³ est un hydrogène, un groupe hydrocarboné aliphatique saturé ayant de 1 à 10 atomes de carbone ou un groupe -(CO)-R⁵ où R⁵ est un groupe hydrocarboné aliphatique saturé ayant de 1 à 5 atomes de carbone; d est un nombre entier de 2 à 200 et e est un nombre entier de 0 à 200, pourvu que d + e soit un nombre allant de 3 à 200, et n est un nombre de 2 à 6, a est une valeur satisfaisant à l'inégalité : 1,0 ≤ a ≤ 2.5, b est une valeur satisfaisant à l'inégalité : 0.001 ≤ b ≤ 1.0 et c est une valeur satisfaisant à l'inégalité : 0.001 ≤ c ≤ 1.0 ;
ou un organohydrogènepolysiloxane représenté par la formule (2) suivante :
R¹ _{f}H_{g}SiO_{(4-f-g)/2} (2)
dans laquelle R¹ a la même signification que dans la formule (1), f est une valeur satisfaisant à l'inégalité : 1.0 ≤ f ≤ 3.0, g est une valeur satisfaisant à l'inégalité : 0.001 ≤ g ≤ 1.5 ;
ou un mélange des organohydrogènepolysiloxanes de formules (1) et (2), et
(II) un polyoxyalkylène représenté par la formule (A) suivante :
CₘH₂ₘO(C₂H₄O)ₕ(C₃H₆O)ᵢCₘH₂ₘ₋₁ (A)
dans laquelle h est un nombre entier allant de 2 à 200, i est un nombre entier allant de 0 à 200, pourvu que h + i soit un nombre allant de 3 à 200, et m est un nombre allant de 2 à 6.
ou un organopolysiloxane représenté par la formule (B) suivante :
R¹ ⱼR⁴ ₖSiO_{(4-j-k)/2} (B)
dans laquelle R¹ a la même signification que dans la formule (1), R⁴ est un groupe hydrocarboné monovalent ayant une liaison aliphatique insaturée en extrémité et contenant 2 à 10 atomes de carbone, j est une valeur satisfaisant à l'inégalité : 1.0 ≤ j ≤ 3.0 et k est une valeur satisfaisant à l'inégalité : 0.001 ≤ k ≤ 1.5,
ou un mélange du polyoxyalkylène de formule (A) et de l'organopolysiloxane de formule (B), où au moins un organohydrogènepolysiloxane de formule (1) ou au moins un polyoxyalkylène de formule (A) est contenu comme élément essentiel de la polymérisation d'addition.

2. Emulsion selon la revendication 1, **caractérisée par le fait que** l'organopolysiloxane est en mélange avec une huile de silicone et/ou un polyol.

3. Emulsion selon la revendication 2, **caractérisée par le fait que** l'huile de silicone a de préférence une viscosité égale ou inférieure à 100 mm²/s à 25°C.

4. Emulsion selon la revendication précédente, **caractérisée par le fait que** l'huile de silicone a une viscosité égale à 6 mm²/s à 25°C.

5. Emulsion selon la revendication précédente, **caractérisée par le fait que** l'huile de silicone est une huile de silicone volatile ou une huile de silicone non volatile ou un mélange des deux.

6. Emulsion selon l'une quelconque des revendications précédentes, **caractérisée en ce que** l'organopolysiloxane est présent en une quantité en matière active allant de 0,1 à 10 % du'poids total de l'émulsion triple.

7. Emulsion selon l'une quelconque des revendications précédentes, **caractérisée en ce que** la phase aqueuse externe contient au moins un tensioactif non ionique ayant un HLB supérieur à 12.

8. Emulsion selon la revendication précédente, **caractérisée en ce que** le tensioactif non ionique est choisi parmi les alcools gras éthoxylés ou éthoxylés/propoxylés à chaine grasse comportant de 12 à 22 atomes de carbone, les stérols éthoxylés, les copolymères blocs d'oxyde d'éthylène et d'oxyde de propylène et leurs mélanges.

9. Emulsion selon l'une quelconque des revendications précédentes, **caractérisée en ce que** l'émulsion primaire contient un tensioactif non ionique de HLB < 8.

10. Emulsion selon la revendication précédente, **caractérisée en ce que** le tensioactif non ionique de HLB<8 est choisi parmi les esters de glycéryle, les esters de sucre, les éthers d'alkyl-polyglucoside et leurs mélanges.

11. Emulsion selon l'une quelconque des revendications précédentes, **caractérisée en ce que** la phase huileuse contient un ou plusieurs corps gras choisis parmi les huiles d'origine animale, les huiles d'origine végétale, les huiles minérales, les huiles synthétiques, les huiles fluorées, les cires, les gommes de silicone, les résines de silicone.

12. Emulsion selon l'une quelconque des revendications précédentes, **caractérisée en ce que** la quantité de corps gras va de 2 à 40 % du poids total de l'émulsion triple.

13. Emulsion selon l'une quelconque des revendications précédentes, **caractérisée en ce que** l'émulsion primaire représente de 5 à 70 % du poids total de l'émulsion triple.

14. Composition topique, **caractérisée en ce qu'**elle contient une émulsion selon l'une quelconque des revendications précédentes et au moins un actif.

15. Composition topique selon la revendication 14, **caractérisée en ce que** l'actif est un actif cosmétique.

16. Composition selon la revendication 15, **caractérisée en ce que** l'actif est choisi parmi les polyols, les vitamines, les bêta-hydroxyacides, les alpha-hydroxyacides, les filtres, les hydratants, les actifs instables en milieu oxydant et leurs mélanges.

17. Composition selon la revendication 15 ou 16, **caractérisée en ce que** l'actif est choisi parmi la vitamine C, la vitamine A, l'urée, la rutine, les enzymes, les extraits naturels, les oligomères procyannidoliques, les caroténoïdes, les acides gras polyinsaturés et leurs mélanges.

18. Composition selon l'une quelconque des revendications 15 à 17, **caractérisée en ce que** l'actif est présent en une concentration allant de 0,01 à 20 % du poids total de la composition.

19. Composition selon l'une quelconque des revendications 15 à 18, **caractérisée en ce qu'**elle comprend au moins un adjuvant lipophile ou hydrophile choisi parmi les conservateurs, les antioxydants, les séquestrants, les solvants, les parfums, les charges, les filtres, les absorbeurs d'odeur, les matières colorantes et les vésicules lipidiques.

20. Utilisation cosmétique de la composition selon l'une quelconque des revendications 15 à 19 pour nettoyer et/ou traiter et/ou protéger la peau, les muqueuses et/ou les fibres kératiniques.

21. Utilisation de la composition selon l'une quelconque des revendications 14 à pour la préparation d'une composition destinée à nettoyer et/ou traiter et/ou protéger la peau et/ou les muqueuses et/ou les fibres kératiniques.

22. Procédé cosmétique pour nettoyer et/ou traiter et/ou protéger la peau et/ou les fibres kératiniques, **caractérisé en ce qu'**il consiste à appliquer sur la peau, les muqueuses et/ou les fibres kératiniques, une composition selon l'une quelconque des revendications 15 à 19.

23. Utilisation d'au moins un organopolysiloxane élastomère partiellement ou totalement réticulé comportant une chaîne polyoxyéthylénée et/ou polyoxypropylénée, pour la stabilisation d'une émulsion triple eau/huile/eau, l'organosiloxane élastomère étant obtenu par polymérisation d'addition des composés (I) et (II) suivants :
(I) un organohydrogènepolysiloxane de formule (1) :
R¹ ₐR² _{b}H_{c}SiO_{(4-a-b-c)/2} (1)
dans laquelle R¹ représente un groupe alkyle, aryle ou aralkyle substitué ou non substitué, comportant de 1 à 18 atomes de carbone, ou un groupe hydrocarboné halogéné ; R² représente un groupe :
-CₙH₂ₙO(C₂H₄O)_{d}(C₃H₆O)ₑR³ (3)
dans lequel R³ est un hydrogène, un groupe hydrocarboné aliphatique saturé ayant de 1 à 10 atomes de carbone ou un groupe -(CO)-R⁵ où R⁵ est un groupe hydrocarboné aliphatique saturé ayant de 1 à 5 atomes de carbone; d est un nombre entier de 2 à 200 et e est un nombre entier de 0 à 200, pourvu que d + e soit un nombre allant de 3 à 200, et n est un nombre de 2 à 6, a est une valeur satisfaisant à l'inégalité : 1.0 ≤ a ≤ 2.5, b est une valeur satisfaisant à l'inégalité : 0.001 ≤ b ≤ 1.0 et c est une valeur satisfaisant à l'inégalité : 0.001 ≤ c ≤ 1.0 ;
ou un organohydrogènepolysiloxane représenté par la formule (2) suivante :
R¹ _{f}H_{g}SiO_{(4-f-g)/2} (2)
dans laquelle R¹ a la même signification que dans la formule (1), f est une valeur satisfaisant à l'inégalité : 1.0 ≤ f ≤ 3.0, g est une valeur satisfaisant à l'inégalité : 0.001 ≤ g ≤ 1.5;
ou un mélange des organohydrogènepolysiloxanes de formules (1) et (2), et
(II) un polyoxyalkylène représenté par la formule (A) suivante :
CₘH₂ₘO(C₂H₄O)ₕ(C₃H₆O)ᵢCₘH₂ₘ₋₁ (A)
dans laquelle h est un nombre entier allant de 2 à 200, i est un nombre entier allant de 0 à 200, pourvu que h + i soit un nombre allant de 3 à 200, et m est un nombre allant de 2 à 6,
ou un organopolysiloxane représenté par la formule (B) suivante :
R¹ ⱼR⁴ ₖSiO_{(4-j-k)/2} (B)
dans laquelle R¹ a la même signification que dans la formule (1), R⁴ est un groupe hydrocarboné monovalent ayant une liaison aliphatique insaturée en extrémité et contenant 2 à 10 atomes de carbone, j est une valeur satisfaisant à l'inégalité : 1.0 ≤ j ≤ 3.0 et k est une valeur satisfaisant à l'inégalité : 0.001 ≤ k ≤ 1.5,
ou un mélange du polyoxyalkylène de formule (A) et de l'organopolysiloxane de formule (B), où au moins un organohydrogènepolysiloxane de formule (1) ou au moins un polyoxyalkylène de formule (A) est contenu comme élément essentiel de la polymérisation d'addition.

## Patentansprüche

1. Dreifache Wasser-in-Öl-in-Wasser-Emulsion, die eine äußere wäßrige Phase und eine primäre W/O-Emulsion mit einer Ölphase und einer inneren wäßrigen Phase enthält, **dadurch gekennzeichnet, daß** die Ölphase mindestens ein ganz oder teilweise vernetztes elastomeres Organopolysiloxan mit polyethoxylierter und/oder polypropoxylierter Kette enthält, das durch Additionspolymerisation der folgenden Verbindungen (I) und (II) hergestellt wird:
(I) Organohydrogenpolysiloxanen der Formel (1):
R¹ ₐ R² _{b}H_{c}SiO_{(4-a-b-c)/2} (1),
worin bedeuten:
R¹ eine substituierte oder unsubstituierte Alkyl-, Aryl- oder Aralkylgruppe, die 1 bis 18 Kohlenstoffatome aufweist, oder eine halogenierte Kohlenwasserstoffgruppe;
R² eine Gruppe:
-CₙH₂ₙO(C₂H₄O)_{d}(C₃H₆O)ₑR³ (3),
worin bedeuten: R³ Wasserstoff, eine gesättigte aliphatische Kohlenwasserstoffgruppe mit 1 bis 10 Kohlenstoffatomen oder eine Gruppe -(CO)-R⁵, wobei R⁵ eine gesättigte aliphatische Kohlenwasserstoffgruppe mit 1 bis 5 Kohlenstoffatomen ist; d eine ganze Zahl von 2 bis 200, e Null oder eine ganze Zahl von 1 bis 200, mit der Maßgabe, daß d + e eine Zahl von 3 bis 200 bedeutet; n eine Zahl von 2 bis 6;
a einen Wert, der die folgende Ungleichung erfüllt:
1,0 ≤ a ≤ 2,5;
b einen Wert, der die folgende Ungleichung erfüllt:
0,001 ≤ b ≤ 1,0; und
c einen Wert, der die folgende Ungleichung erfüllt:
0,001 ≤ c ≤ 1,0;
oder Organohydrogenpolysiloxanen der folgenden Formel (2):
R¹ _{f}H_{g}SiO_{(4-f-g)/2} (2),
worin R¹ die oben für Formel (1) angegebenen Bedeutungen aufweist, f ein Wert ist, der die folgende Ungleichung erfüllt: 1,0 ≤ f ≤ 3,0; und g ein Wert ist, der die folgende Ungleichung erfüllt: 0,001 ≤ g ≤ 1,5;
oder Gemischen von Organohydrogenpolysiloxanen der Formeln (1) und (2), und
(II) Polyalkylenoxiden der folgenden Formel (A):
CₘH₂ₘO(C₂H₄O)ₕ(C₃H₆O)ᵢCₘH₂ₘ₋₁ (A),
worin bedeuten: h eine ganze Zahl von 2 bis 200, i O oder eine ganze Zahl von 1 bis 200, mit der Maßgabe, daß h + i eine Zahl von 3 bis 200 ist, und m eine Zahl von 2 bis 6;
oder Organopolysiloxanen der folgenden Formel (B):
R¹ ⱼR⁴ ₖSiO_{(4-j-k)/2} (B),
wobei R¹ die für Formel (1) angegebenen Bedeutungen aufweist, R⁴ eine einwertige Kohlenwasserstoffgruppe mit 2 bis 10 Kohlenstoffatomen ist, die am Ende eine Doppelbindung aufweist, j einen Wert bedeutet, der die folgende Ungleichung erfüllt: 1,0 ≤ j ≤ 3,0; und k ein Wert ist, der die folgende Ungleichung erfüllt: 0,001 **≤** k ≤ 1,5;
oder Gemischen von Polyalkylenoxiden der Formel (A) und Organopolysiloxanen der Formel (B), wobei mindestens ein Organohydrogenpolysiloxan der Formel (1) oder mindestens ein Polyalkylenoxid der Formel (A) als wesentliches Element der Additionspolymerisation enthalten ist.

2. Emulsion nach Anspruch 1, **dadurch gekennzeichnet, daß** das Organopolysiloxan im Gemisch mit einem Siliconöl und/oder Polyol vorliegt.

3. Emulsion nach Anspruch 2, **dadurch gekennzeichnet, daß** das Siliconöl bei 25 °C vorzugsweise eine Viskosität von höchstens 100 mm²/s aufweist.

4. Emulsion nach dem vorhergehenden Anspruch, **dadurch gekennzeichnet, daß** das Siliconöl bei 25 °C eine Viskosität von 6 mm²/s aufweist.

5. Emulsion nach dem vorhergehenden Anspruch, **dadurch gekennzeichnet, daß** das Siliconöl ein flüchtiges Siliconöl oder ein nicht flüchtiges Siliconöl oder ein Gemisch dieser beiden Öle ist.

6. Emulsion nach einem der vorhergehenden Ansprüche, **dadurch gekennzeichnet, daß** das Organopolysiloxan in einer Wirkstoffmenge von 0,1 bis 10 % des Gesamtgewichts der dreifachen Emulsion vorliegt.

7. Emulsion nach einem der vorhergehenden Ansprüche, **dadurch gekennzeichnet, daß** die äußere wäßrige Phase mindestens einen nichtionischen grenzflächenaktiven Stoff mit einem HLB-Wert über 12 enthält.

8. Emulsion nach dem vorhergehenden Anspruch, **dadurch gekennzeichnet, daß** der nichtionische grenzflächenaktive Stoff unter den ethoxylierten oder ethoxylierten/propoxylierten Fettalkoholen mit C₁₂₋₂₂-Fettkette, den ethoxylierten Sterinen, Blockcopolymeren von Ethylenoxid und Propylenoxid und deren Gemischen ausgewählt ist.

9. Emulsion nach einem der vorhergehenden Ansprüche, **dadurch gekennzeichnet, daß** die primäre Emulsion einen nichtionischen grenzflächenaktiven Stoff mit einem HLB-Wert < 8 enthält.

10. Emulsion nach dem vorhergehenden Anspruch, **dadurch gekennzeichnet, daß** der nichtionische grenzflächenaktive Stoff mit einem HLB-Wert < 8 unter den Glycerylestern, Zuckerestern, Alkylpolyglucosidethern und deren Gemischen ausgewählt ist.

11. Emulsion nach einem der vorhergehenden Ansprüche, **dadurch gekennzeichnet, daß** die Ölphase eine oder mehrere Fettsubstanzen enthält, die unter den Ölen pflanzlichen Ursprungs, Mineralölen, synthetischen Ölen, fluorierten Ölen, Wachsen, Silicongummis und Siliconharzen ausgewählt sind.

12. Emulsion nach einem der vorhergehenden Ansprüche, **dadurch gekennzeichnet, daß** der Mengenanteil der Fettsubstanzen im Bereich von 2 bis 40 % des Gesamtgewichts der dreifachen Emulsion liegt.

13. Emulsion nach einem der vorhergehenden Ansprüche, **dadurch gekennzeichnet, daß** die primäre Emulsion 5 bis 70 % des Gesamtgewichts der dreifachen Emulsion ausmacht.

14. Topische Zusammensetzung, **dadurch gekennzeichnet, daß** sie eine Emulsion nach einem der vorhergehenden Ansprüche und mindestens einen Wirkstoff enthält.

15. Topische Zusammensetzung nach Anspruch 14, **dadurch gekennzeichnet, daß** der Wirkstoff ein kosmetischer Wirkstoff ist.

16. Zusammensetzung nach Anspruch 15, **dadurch gekennzeichnet, daß** der Wirkstoff unter den Polyolen, Vitaminen, β-Hydroxysäuren, α-Hydroxysäuren, Filtern, Hydratisierungsmitteln, in oxidierenden Medien instabilen Wirkstoffen und deren Gemischen ausgewählt ist.

17. Zusammensetzung nach Anspruch 15 oder 16, **dadurch gekennzeichnet, daß** der Wirkstoff unter Vitamin C, Vitamin A, Harnstoff, Rutin, Enzymen, natürlichen Extrakten Procyanidin-Oligomeren, Carotinoiden, mehrfach ungesättigten Fettsäuren und deren Gemischen ausgewählt ist.

18. Zusammensetzung nach einem der Ansprüche 15 bis 17, **dadurch gekennzeichnet, daß** der Wirkstoff in einer Konzentration von 0,01 bis 20 % des Gesamtgewichts der Zusammensetzung vorliegt.

19. Zusammensetzung nach einem der Ansprüche 15 bis 18, **dadurch gekennzeichnet, daß** sie mindestens einen lipophilen oder hydrophilen Zusatzstoff enthält, der unter den Konservierungsmitteln, Antioxidantien, Maskierungsmitteln, Lösemitteln, Parfums, Füllstoffen, Filtern, Geruchsabsorbern, Farbmitteln und Lipidvesikeln ausgewählt ist.

20. Kosmetische Verwendung der Zusammensetzung nach einem der Ansprüche 15 bis 19 zur Reinigung und/oder zur Behandlung und/oder zum Schutz der Haut, der Schleimhäute und/oder der Keratinfasern.

21. Verwendung der Zusammensetzung nach einem der Ansprüche 14 bis 19 zur Herstellung einer Zusammensetzung, die zur Reinigung und/oder zur Behandlung und/oder zum Schutz der Haut und/oder der Schleimhäute und/oder der Keratinfasern vorgesehen ist.

22. Kosmetisches Verfahren zur Reinigung und/oder zur Behandlung und/oder zum Schutz der Haut, der Schleimhäute und/oder der Keratinfasern, **dadurch gekennzeichnet, daß** es darin besteht, auf die Haut, die Schleimhäute und/oder die Keratinfasern eine Zusammensetzung nach einem der Ansprüche 15 bis 19 aufzutragen.

23. Verwendung mindestens eines ganz oder teilweise vernetzten elastomeren Organopolysiloxans mit polyethoxylierter und/oder polypropoxylierter Kette zur Stabilisierung einer dreifachen W/O/W-Emulsion, wobei das Organopolysiloxan durch Additionspolymerisation der folgenden Verbindungen (I) und (II) hergestellt wird:
(I) Organohydrogenpolysiloxanen der Formel (1):
R¹ ₐ R² _{b}H_{c}SiO_{(4-a-b-c)/2} (1),
worin bedeuten:
R¹ eine substituierte oder unsubstituierte Alkyl-, Aryl- oder Aralkylgruppe, die 1 bis 18 Kohlenstoffatome aufweist, oder eine halogenierte Kohlenwasserstoffgruppe;
R² eine Gruppe:
-CₙH₂ₙO(C₂H₄O)_{d}(C₃H₆O)ₑR³ (3),
worin bedeuten: R³ Wasserstoff, eine gesättigte aliphatische Kohlenwasserstoffgruppe mit 1 bis 10 Kohlenstoffatomen oder eine Kohlenwasserstoffgruppe -(CO)-R⁵, wobei R⁵ eine gesättigte aliphatische Gruppe mit 1 bis 5 Kohlenstoffatomen ist; d eine ganze Zahl von 2 bis 200, e Null oder eine ganze Zahl von 1 bis 200, mit der Maßgabe, daß d + e eine Zahl von 3 bis 200 bedeutet; n eine Zahl von 2 bis 6;
a einen Wert, der die folgende Ungleichung erfüllt:
1,0 ≤ a ≤ 2,5;
b einen Wert, der die folgende Ungleichung erfüllt:
0,001 ≤ b ≤ 1,0;
c einen Wert, der die folgende Ungleichung erfüllt:
0,001 ≤ c ≤ 1,0;
oder Organohydrogenpolysiloxanen der folgenden Formel (2):
R¹ _{f} H_{g}SiO_{(4-f-g)/2} (2),
worin R¹ die für Formel (1) angegebenen Bedeutungen aufweist, f ein Wert ist, der die folgende Ungleichung erfüllt: 1,0 ≤ f ≤ 3,0; und g ein Wert ist, der die folgende Ungleichung erfüllt: 0,001 ≤ g ≤ 1,5;
oder Gemischen von Organohydrogenpolysiloxanen der Formeln (1) und (2), und
(II) Polyalkylenoxiden der folgenden Formel (A):
CₘH₂ₘO(C₂H₄O)ₕ(C₃H₆O)ᵢCₘH₂ₘ₋₁ (A),
worin bedeuten: h eine ganze Zahl von 2 bis 200, i 0 oder eine ganze Zahl von 1 bis 200, mit der Maßgabe, daß h + i eine Zahl von 3 bis 200 ist, und m eine Zahl von 2 bis 6;
oder Organopolysiloxanen der folgenden Formel (B):
R¹ ⱼR⁴ ₖSiO_{(4-j-k)/2} (B),
wobei R¹ die für Formel (1) angegebenen Bedeutungen aufweist, R⁴ eine einwertige Kohlenwasserstoffgruppe mit 2 bis 10 Kohlenstoffatomen ist, die am Ende eine aliphatische ungesättigte Bindung aufweist, j einen Wert bedeutet, der die folgende Ungleichung erfüllt: 1,0 ≤ j ≤ 3,0; und k ein Wert ist, der die folgende Ungleichung erfüllt: 0,001 ≤ k ≤ 1,5;
oder Gemischen von Polyalkylenoxiden der Formel (A) und Organopolysiloxanen der Formel (B), wobei als wesentliches Element für die Additionspolymerisation mindestens ein Organohydrogenpolysiloxan der Formel (1) oder mindestens ein Polyalkylenoxid der Formel (A) enthalten ist.

## Claims

1. water/oil/water triple emulsion comprising an aqueous external phase and a W/O primary emulsion comprising an oily phase and an aqueous internal phase, **characterized in that** the oily phase comprises at least one partially or completely crosslinked organopolysiloxane elastomer comprising, a polyoxyethylenated and/or polyoxypropylenated chain obtained by addition polymerization of the following compounds (I) and (II):
(I) an organohydropolysiloxane of formula (1):
R¹ ₐR² _{b}H_{c}SiO_{(4-a-b-c)/2} (1)
in which R¹ represents a substituted or unsubstituted alkyl, aryl or aralkyl group comprising from 1 to 18 carbon atoms or a halogenated hydrocarbon-comprising group; R² represents a group:
-CₙH₂ₙO(C₂H₄O)_{d}(C₃H₆O)ₑR³ (3)
in which R³ is a hydrogen, a saturated aliphatic hydrocarbon-comprising group having from 1 to 10 carbon atoms or a -(CO)-R⁵ group where R⁵ is a saturated aliphatic hydrocarbon-comprising group having from 1 to 5 carbon atoms; d is an integer from 2 to 200 and e is an integer from 0 to 200, provided that d + e is a number ranging from 3 to 200, and n is a number from 2 to 6, a is a value satisfying the inequality: 1.0 ≤ a ≤ 2.5, b is a value satisfying the inequality: 0.001 ≤ b ≤ 1.0 and c is a value satisfying the inequality: 0.001 ≤ c ≤ 1.0;
or an organohydropolysiloxane represented by the following formula (2):
R¹ _{f}H_{g}SiO_{(4-f-g)/2} (2)
in which R¹ has the same meaning as in the formula (1), f is a value satisfying the inequality: 1,0 ≤ f ≤ 3.0 and g is a value satisfying the inequality: 0.001 ≤ g ≤ 1.5;
or a mixture of the organohydropolysiloxanes of formulae (1) and (2), and
(II) a polyoxyalkylene represented by the following formula (A):
CₘH₂ₘO(C₂H₄O)ₕ(C₃H₆O)ᵢCₘH₂ₘ₋₁ (A)
in which h is an integer ranging from 2 to 200, i is an integer ranging from 0 to 200, provided that h + i is a number ranging from 3 to 200, and m is a number ranging from 2 to 6,
or an organopolysiloxane represented by the following formula (B):
R¹ ⱼR⁴ ₖSiO_{(4-j-k)/2} (B)
in which R¹ has the same meaning as in the formula (1), R⁴ is a monovalent hydrocarbon-comprising group having an unsaturated aliphatic bond at the end and comprising 2 to 10 carbon atoms, j is a value satisfying the inequality: 1.0 ≤ j ≤ 3.0 and k is a value satisfying the inequality: 0.001 ≤ k ≤ 1.5,
or a mixture of the polyoxyalkylene of formula (A) and of the organopolysiloxane of formula (B), where at least one organohydropolysiloxane of formula (1) or at least one polyoxyalkylene of formula (A) is present as essential component of the addition polymerization.

2. Emulsion according to Claim 1, **characterized in that** the organopolysiloxane is mixed with a silicone oil and/or a polyol.

3. Emulsion according to Claim 2, **characterized in that** the silicone oil preferably has a viscosity equal to or less than 100 mm²/s at 25°C.

4. Emulsion according to the preceding claim, **characterized in that** the silicone oil has a viscosity equal to 6 mm²/s at 25°C.

5. Emulsion according to the preceding claim, **characterized in that** the silicone oil is a volatile silicone oil or a nonvolatile silicone oil or a mixture of the two.

6. Emulsion according to any one of the preceding claims, **characterized in that** the organopolysiloxane is present in an amount of active material ranging from 0.1 to 10% of the total weight of the triple emulsion.

7. Emulsion according to any one of the preceding claims, **characterized in that** the aqueous external phase comprises at least one nonionic surfactant having an HLB of greater than 12.

8. Emulsion according to the preceding claim, **characterized in that** the nonionic surfactant is chosen from ethoxylated or ethoxylated/propoxylated fatty alcohols with a fatty chain comprising from 12 to 22 carbon atoms, ethoxylated sterols, block copolymers of ethylene oxide and propylene oxide, and their mixtures.

9. Emulsion according to any one of the preceding claims, **characterized in that** the primary emulsion comprises a nonionic surfactant with an HLB < 8.

10. Emulsion according to the preceding claim, **characterized in that** the nonionic surfactant with an HLB < 8 is chosen from glyceryl esters, sugar esters, alkylpolyglucoside ethers and their mixtures.

11. Emulsion according to any one of the preceding claims, **characterized in that** the oily phase comprises one or more fatty substances chosen from oils of animal origin, oils of vegetable origin, mineral oils, synthetic oils, fluorinated oils, waxes, silicone gums or silicone resins.

12. Emulsion according to any one of the preceding claims, **characterized in that** the amount of fatty substances ranges from 2 to 40% of the total weight of the triple emulsion.

13. Emulsion according to any one of the preceding claims, **characterized in that** the primary emulsion represents from 5 to 70% of the total weight of the triple emulsion.

14. Topical composition, **characterized in that** it comprises an emulsion according to any one of the preceding claims and at least one active principle.

15. Topical composition according to Claim 14, **characterized in that** the active principle is a cosmetic active principle.

16. Composition according to Claim 15, **characterized in that** the active principle is chosen from polyols, vitamins, beta-hydroxy acids, alpha-hydroxy acids, screening agents, moisturisers, active principles which are unstable in an oxidizing medium, and their mixtures.

17. Composition according to Claim 15 or 16, **characterized in that** the active principle is chosen from vitamin C, vitamin A, urea, rutin, enzymes, natural extracts, procyanidol oligomers, carotenoids; polyunsaturated fatty acids and their mixtures.

18. Composition according to any one of Claims 15 to 17, **characterized in that** the active principle is present in a concentration ranging from 0.01 to 20% of the total weight of the composition.

19. Composition according to any one of Claims 15 to 18, **characterized in that** it comprises at least one lipophilic or hydrophilic adjuvant chosen from preservatives, antioxidants, sequestering agents, solvents, fragrances, fillers, screening agents, odour absorbers, colouring materials and lipid vesicles,

20. Cosmetic use of the composition according to any one of Claims 15 to 19 for cleansing and/or treating and/or protecting the skin, mucous membranes and/or keratinous fibres.

21. Use of the composition according to any one of Claims 14 to 19 for the preparation of a composition intended to cleanse and/or treat and/or protect the skin and/or mucous membranes and/or keratinous fibres.

22. Cosmetic process for cleansing and/or treating and/or protecting the skin and/or keratinous fibres, **characterized in that** it consists in applying a composition according to any one of Claims 15 to 19 to the skin, mucous membranes and/or keratinous fibres.

23. Use of at least one partially or completely crosslinked organopolysiloxane elastomer comprising a polyoxyethylenated and/or polyoxypropylenated chain for the stabilization of a water/oil/water triple emulsion, the organopolysiloxane elastomer being obtained by addition polymerization of the following compounds (I) and (II) ;
(I) an organohydropolysiloxane of formula (1) :
R¹ ₐR² _{b}H_{c}SiO_{(4-a-b-c)/2} (1)
in which R¹ represents a substituted or unsubstituted alkyl, aryl or aralkyl group comprising from 1 to 18 carbon atoms or a halogenated hydrocarbon-comprising group; R² represents a group:
-CₙH₂ₙO(C₂H₄O)_{d}(C₃H₆O)ₑR³ (3)
in which R³ is a hydrogen, a saturated aliphatic hydrocarbon-comprising group having from 1 to 10 carbon *atoms* or a -(CO)-R⁵ group where R⁵ is a saturated aliphatic hydrocarbon-comprising group having from 1 to 5 carbon atoms; d is an integer from 2 to 200 and e is an integer from 0 to 200, provided that d + e is a number ranging from 3 to 200, and n is a number from 2 to 6, a is a value satisfying the inequality: 1.0 ≤ a ≤ 2.5, b is a value satisfying the inequality: 0.001 ≤ b ≤ 1.0 and c is a value satisfying the inequality: 0.001 ≤ c ≤ 1.0;
or an organohydropolysiloxane represented by the following formula (2):
R¹ _{f}H_{g}SiO_{(4-f-g)/2} (2)
in which R¹ has the same meaning as in the formula (1), f is a value satisfying the inequality: 1.0 ≤ f ≤ 3.0 and g is a value satisfying the inequality: 0.001 ≤ g ≤ 1.5;
or a mixture of the organohydropolysiloxanes of formulae (1) and (2), and
(II) a polyoxyalkylene represented by the following formula (A):
CₘH₂ₘO(C₂H₄O)ₕ(C₃H₆O)ᵢCₘH₂ₘ₋₁ (A)
in which h is an integer ranging from 2 to 200, i is an integer ranging from 0 to 200, provided that h + i is a number ranging from 3 to 200, and m is a number ranging from 2 to 6,
or an organopolysiloxane represented by the following formula (B):
R¹ ⱼR⁴ ₖSiO_{(4-j-k)/2} (B)
in which R¹ has the same meaning as in the formula (1), R⁴ is a monovalent hydrocarbon-comprising group having an unsaturated aliphatic bond at the end and comprising 2 to 10 carbon atoms, j is a value satisfying the inequality; 1.0 ≤ j ≤ 3.0 and k is a value satisfying the inequality; 0.001 ≤ k ≤ 1.5,
or a mixture of the polyoxyalkylene of formula (A) and of the organopolysiloxane of formula (B), where at least one organohydropolysiloxane of formula (1) or at least one polyoxyalkylene of formula (A) is present as essential component of the addition polymerization.
